# EUROPÄISCHE PATENTSCHRIFT

(11) **EP 1 317 295 B1**
(45) Veröffentlichungstag und Bekanntmachung des Hinweises auf die Patenterteilung: **03.01.2007**
(21) Anmeldenummer: 01969363.9
(22) Anmeldetag: 10.07.2001
(51) Int. Cl.: A61M 1/10

(54) **INTRAKARDIALE BLUTPUMPE**
INTRACARDIAC BLOOD PUMP
POMPE A SANG INTRACARDIAQUE

(30) Priorität: 18.08.2000 DE 10040403
(43) Veröffentlichungstag der Anmeldung: 11.06.2003
(73) Patentinhaber: Abiomed Europe GmbH, 52074 Aachen (DE)
(72) Erfinder: SIESS, Thorsten, 52146 Würselen (DE)
(74) Vertreter: Selting, Günther
(86) Internationale Anmeldenummer: PCT/EP2001/007953
(87) Internationale Veröffentlichungsnummer: WO 2002/015963

(56) Entgegenhaltungen:
- WO-A-97/37696
- DE-C- 19 821 307
- US-A- 5 275 580
- US-A- 5 964 694

## Beschreibung

Die Erfindung betrifft eine intrakardiale Blutpumpe mit einem einen Motor enthaltenden Antriebsteil und einem Pumpenteil, der ein von dem Motor angetriebenes Pumpenrad enthält, wobei zwischen dem Antriebsteil und dem Pumpenteil mindestens eine seitliche Durchströmöffnung vorgesehen ist.

Eine intrakardiale Blutpumpe ist eine Blutpumpe, die mindestens teilweise in das Herz eingeführt wird, um Blut aus dem Herzen in eine Arterie zu fördern, wobei die Pumpe durch eine Operationsöffnung des Herzens hindurchragen kann. Solche intrakardialen Blutpumpen haben einen maximalen Außendurchmesser von etwa 10 - 15 mm. Eine spezielle Form intrakardialer Blutpumpen sind intravasale Blutpumpen. Diese werden durch das Gefäßsystem des Patienten hindurch in das Herz eingeführt, wobei die Inzisionsstelle sich im Abstand vom Herzen befindet. Intravasale Blutpumpen haben einen Durchmesser von maximal etwa 8 mm und eine rigide Länge von maximal 35 mm.

Intravasale Blutpumpen sind bekannt aus WO 94/09835 (Jarvik) und EP 0 764 448 A2 (Jarvik). Eine intravasale Blutpumpe, bei der der Außendurchmesser nur etwa 6 mm beträgt, ist beschrieben in WO 97/37696.

Eine intrakardiale Blutpumpe, von der der Oberbegriff des Patentanspruchs 1 ausgeht, ist beschrieben in DE 198 21 307 C1. Diese Schrift beschreibt den Rechtsherzbetrieb einer intrakardialen Blutpumpe. Die Blutpumpe wird hierbei durch die obere Hohlvene hindurch in dem rechten Atrium plaziert und eine mit dem Pumpenauslass verbundene flexible Kanüle erstreckt sich durch die Tricuspidalklappe in den rechten Ventrikel und von dort durch die Pulmonalklappe in die Pulmonalarterie. Die Pumpe fördert vom rechten Atrium in die zur Lunge führende Pulmonalarterie. Der Pumpenauslass ist mit einem flexiblen Pumpenschlauch verbunden, an dessen Ende sich ein Ballon und/oder ein Segel befindet. Die Durchströmöffnung des Pumpengehäuses liegt frei und ist nicht bedeckt.

US-A-5,964,694 beschreibt neben anderen Ausführungsbeispielen auch eine intravasale Blutpumpe, bei der zwischen Antriebsteil und Pumpenteil seitliche Durchströmöffnungen vorgesehen sind. An dem Pumpenteil ist ein Schirm befestigt, der sich an die Gefäßwand des betreffenden Blutgefäßes anlegen und eine seitliche Umströmung des Pumpenteils verhindern soll. Der Schirm bildet eine Kappe mit einer schräg vom Pumpenteil abstehenden Wand. In Längsrichtung erstreckt sich diese Kappe nur über einen geringen Bruchteil der Länge der Durchströmöffnung.

Bei einer intravasalen Blutpumpe ist eine Förderleistung von ca. 4,5 1/min. bei physiologischen Drücken (60 - 80 mm Hg) erforderlich. Diese hohe Förderleistung führt zu hohen Strömungsgeschwindigkeiten in der seitlichen Einlassöffnung zwischen Antriebsteil und Pumpenteil, mit der Folge, dass die Blutpumpe einen starken Sog erzeugt. Dadurch kann die Blutpumpe sich an der relativ dünnen Wand des rechten Atriums festsaugen. Ein solches Festsaugen hat einerseits zur Folge, dass die Einströmöffnung teilweise verschlossen wird und der erforderliche Blutstrom nicht erreicht werden kann, und zum anderen besteht die Gefahr der Verletzung der Wand des Atriums. Des weiteren kann es vorkommen, dass Gewebeteile des Klappenapparats in die Pumpe eingesaugt werden und diese hydraulisch versperren oder mechanisch blocken. Der Funktionsverlust einer zur Herzunterstützung dienenden Pumpe kann lebensgefährliche Folgen haben.

Der vorliegenden Erfindung liegt die Aufgabe zugrunde, eine intrakardiale Blutpumpe zu schaffen, die einen störungsfreien Dauerbetrieb ohne die Gefahr des Festsaugens oder Einsaugens von Körpermaterie ermöglicht.

Die Lösung dieser Aufgabe erfolgt erfindungsgemäß mit den im Anspruch 1 angegebenen Merkmalen. Hiernach ist die zwischen Antriebsteil und Pumpenteil vorgesehene Durchströmöffnung mit einem Schirm bedeckt, der sich über die gesamte Länge der Durchströmöffnung erstreckt und den die Durchströmöffnung passierenden Blutstrom umlenkt. Der Blutstrom passiert die Durchströmöffnung in radialer Richtung in Bezug auf die Längsachse der Blutpumpe, jedoch bewirkt dieser Blutstrom keinen radialen Druck oder Sog. Vielmehr wird der Druck oder Sog von dem Schirm in eine axiale Richtung bezogen auf die Längsachse der Blutpumpe umgeleitet.

Die Blutpumpe kann je nach Drehrichtung des Motors und Ausformung des Pumpenrades mit unterschiedlichen Förderrichtungen betrieben werden. Die Durchströmöffnung zwischen Antriebsteil und Pumpenteil ist in einem Fall eine Einströmöffnung und im anderen Fall eine Ausströmöffnung. In beiden Fällen wirkt sich der Schirm, der im Abstand über der Durchtrittsöffnung angeordnet ist, vorteilhaft auf das Betriebsverhalten aus. Wenn die Durchströmöffnung als Einströmöffnung wirkt, verhindert der Schirm das Festsaugen der Pumpe an externen Teilen dadurch, dass die Einlassöffnung vergrößert und räumlich verteilt wird. Wenn die Blutpumpe derart betrieben wird, dass die Durchströmöffnung die Ausströmöffnung bildet, bewirkt der Schirm eine wirkungsmäßige Verkürzung der Baulänge der Blutpumpe, wie später noch erläutert wird. Dies ermöglicht es, die Pumpe in der Pulmonalarterie zu plazieren, ohne dass die Gefahr von Kurzschlussströmungen durch die Pulmonalklappe besteht.

Ein anderer Aspekt der Erfindung besteht darin, dass an dem Pumpenteil ein Drucksensor vorgesehen sein kann, der den Druck außerhalb und oder innerhalb des Pumpenteils misst und anhand des Messwertes die Förderleistung der Pumpe bestimmt und ferner ungewöhnliche Betriebsbedingungen, wie z.B. einen Leitungsverschluss feststellt und signalisiert. Ein derartiger Sensor wird durch den Schirm überdeckt, so dass nicht die Gefahr besteht, dass der Sensor unter dem Einfluss der Strömungswirkungen gegen eine Wand gedrückt wird und dadurch falsche Druckwerte liefert. Der freiliegend unter dem Schirm angeordnete Sensor kann nicht von dem Ansauggebiet und dem tatsächlichen Ansaugdruck getrennt werden. Wenn ein Ansaugen erfolgt, wird dieses durch den Sensor miterkannt.

Im Folgenden werden unter Bezugnahme auf die Zeichnungen Ausführungsbeispiele der Erfindung näher erläutert.

Es zeigen:
- Fig. 1: eine erste Ausführungsform der Blutpumpe mit vorwärts gerichteter Förderrichtung als intravasale Rechtsherzpumpe im Betrieb in einem Herzen,
- Fig. 2: eine vergrößerte Darstellung der Blutpumpe nach Fig. 1, teilweise aufgebrochen,
- Fig. 3: eine Seitenansicht der Blutpumpe nach Fig. 1,
- Fig. 4: einen Schnitt entlang der Linie IV-IV von Fig. 3,
- Fig. 5: eine zweite Ausführungsform der Blutpumpe mit rückwärts gerichteter Förderrichtung bei Förderung vom rechten Ventrikel in die Pulmonalarterie und
- Fig. 6: eine vergrößerte Darstellung der Blutpumpe nach Fig. 5, teilweise geschnitten.

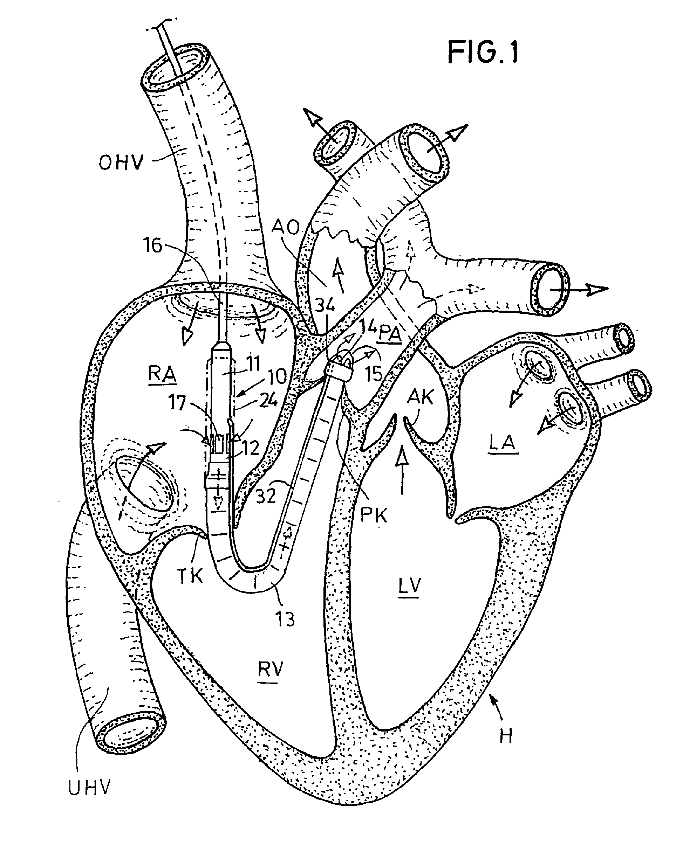

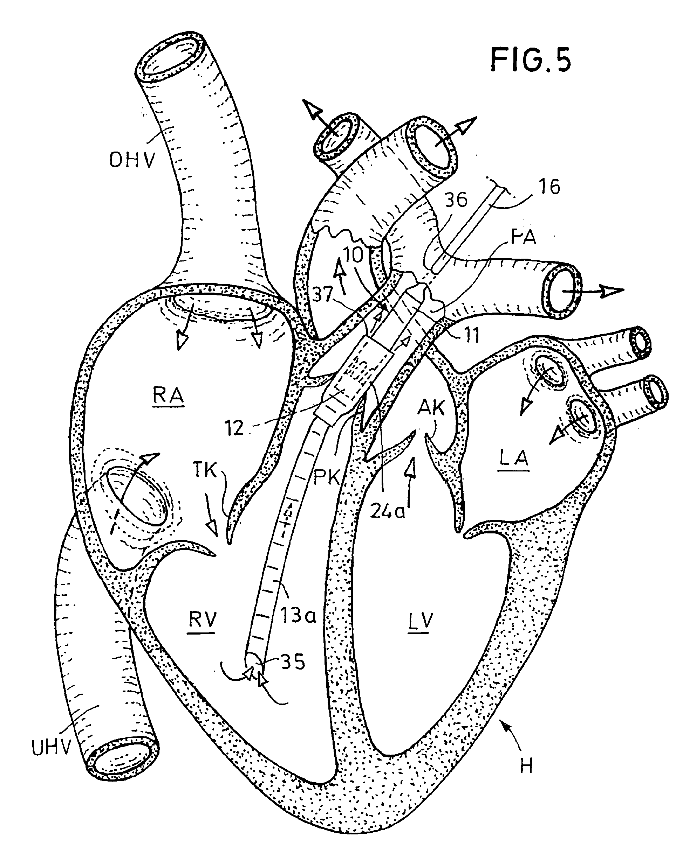

In den Fign. 1 und 5 ist ein Querschnitt durch ein menschliches Herz H dargestellt. In das rechte Atrium RA münden die untere Hohlvene UHV (vena cava inferior) und die obere Hohlvene OHV (vena cava superior). Zwischen dem rechten Atrium RA und dem rechten Ventrikel RV befindet sich die Tricuspidalklappe TK. Zwischen dem rechten Ventrikel RV und der Pulmonalarterie PA befindet sich die Pulmonalklappe PK. Von der Pulmonalarterie PA fließt das Blut über eine Verzweigung zur linken und rechten Lunge und von dort zurück in das linke Atrium LA und zum linken Ventrikel LV. Zwischen dem linken Ventrikel LV und der Aorta AO befindet sich die Aortenklappe AK.

Die Blutpumpe 10 ist eine intravasale Blutpumpe, also eine intrakardiale Blutpumpe, die durch das Blutgefäßsystem eines Patienten geschoben werden kann, um bis in das Herz hinein vorzudringen. Der Außendurchmesser ist an keiner Stelle größer als 10 mm. Die Blutpumpe 10 weist einen Antriebsteil 11, einen Pumpenteil 12 und eine sich von dem Pumpenteil 12 aus erstreckende flexible Kanüle 13 auf, an deren Ende sich eine Austrittsöffnung 14 befindet, welche von einem segelartigen Zugelement 15 überspannt wird. Von dem rückwärtigen Ende des Antriebsteils 11 erstreckt sich ein Katheter 16, welcher ein (nicht dargestelltes) Lumen für einen Führungsdraht aufweist und durch den sich elektrische Leitungen für die Stromversorgung des im Antriebsteil 11 enthaltenen Motors erstrecken.

Zwischen dem Antriebsteil 11 und dem Pumpenteil 12 befinden sich Durchströmöffnungen 17, bei denen es sich hier um Einströmöffnungen handelt, durch welche das Blut in den Pumpenteil 12 strömt, um anschließend durch die Kanüle 13 hindurchgepumpt zu werden.

Die Kanüle 13 kann vorübergehend gestreckt werden, so dass sie durch die obere Hohlvene OHV hindurchgeschoben werden kann. Sie ist so vorgespannt, dass sie im entspannten Zustand die in Fig. 1 dargestellte U-förmige Gestalt annimmt, bei der sie von dem Pumpenteil unter Beschreibung eines Bogens von mehr als 90° zurückgebogen ist, um mit der Austrittsöffnung 14 in der Pulmonalarterie PA plaziert zu werden, während sich die Durchströmöffnung 17 im rechten Atrium RA befindet.

Die Blutpumpe entspricht in ihrer Bauweise grundsätzlich derjenigen von WO 97/37696, so dass ihr interner Aufbau hier nicht mehr detailliert erläutert wird.

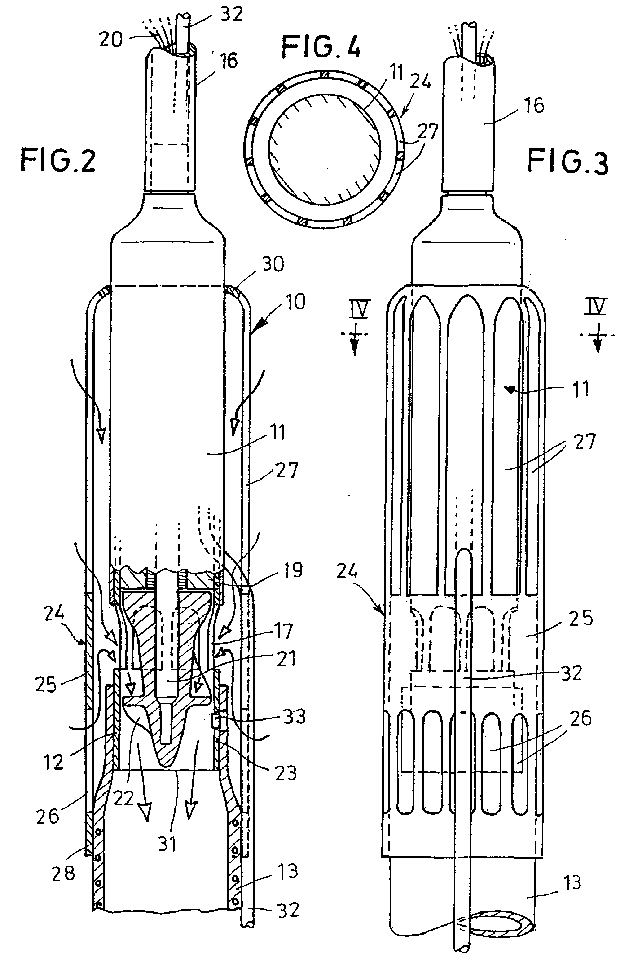

In den Fign. 2 - 4 ist der Grundaufbau der Blutpumpe 10 erkennbar. Der Antriebsteil weist..ein langgestrecktes zylindrisches Gehäuse auf, in welchem sich der elektrische Motor 19 befindet. Der Motor 19 wird über elektrische Leitungen 20, die durch den Katheter 16 verlaufen, von einer extrakorporalen Stromquelle aus mit Strom versorgt. Die Leitungen 20 sind an ein Steuergerät angeschlossen, welches den Motor 19 derart regelt, dass eine gewünschte Pumpleistung erhalten wird. Auf einer Welle 21 des Motors 19 sitzt ein Pumpenrad 22, welches im Pumpenteil 12 rotiert und das Blut axial antreibt. Der Pumpenteil weist einen zylindrischen Pumpenring 23 auf, in dem das Pumpenrad 22 angeordnet ist. Zwischen dem Antriebsteil und dem Pumpenteil 12 befinden sich zahlreiche umfangsmäßig verteilt angeordnete Durchströmöffnungen 17, die bei diesem Ausführungsbeispiel Einlassöffnungen sind. An den Pumpenring 23 schließt sich die Kanüle 13 an. Die Kanüle 13 ist ein flexibler Schlauch aus Polyurethan mit einer elastischen Ringverstärkung.

Damit die Blutpumpe durch die Durchströmöffnungen 17 nicht Fremdmaterial ansaugt oder sich an Wänden nicht festsaugt, ist ein Schirm 24 vorgesehen, der die Durchströmöffnungen 17 mindestens teilweise bedeckt und eine Umlenkung des die Durchströmöffnungen passierenden Blutstromes bewirkt. Der Schirm 24 hat eine zylindrische geschlossene Wand 25, welche die Durchströmöffnungen 17, über deren gesamte Länge überdeckt, und einen vorderen Bereich mit langgestreckten Schlitzen 26 sowie einen hinteren Bereich mit langgestreckten Schlitzen 27. Der Schirm 24 stützt sich mit seinem vorderen Ende 28 auf der Kanüle 13 bzw. dem Pumpenring 23 ab und mit seinem einwärts gebogenen rückwärtigen Ende 30 an dem Antriebsteil 11, das er umschließt. Unter dem Schirm 24 verläuft ein Strömungsweg im Wesentlichen entlang der gesamten Länge des Antriebsteils 11, so dass dieser durch das entlangströmende Blut gekühlt wird. Die Blutpumpe der Fign. 2 und 3 fördert in Vorwärtsrichtung, d. h. sie saugt durch die Durchströmöffnungen 17 an und fördert zu einer Endöffnung 31 am Ende des Pumpenteils 12 bzw. am Übergang des Pumpenteils 12 zu der Kanüle 13. Die geschlossene Wand 25 des Schirms 24 bewirkt die Verteilung der Ansaugwirkung auf die langgestreckten Einlassschlitze 26 und 27, so dass örtliche Saugspitzen vermieden werden.

An dem Pumpenring 23 ist ein Drucksensor 33 angebracht. Dieser Drucksensor ist ein Differenzdrucksensor, der die Differenz zwischen den Drücken außerhalb des Pumpenrings 23 und innerhalb des Pumpenrings ermittelt. Der Drucksensor 33 ist über (nicht dargestellte) Leitungen mit den durch den Katheter 16 hindurchführenden Leitungen verbunden. Er liefert an das extrakorporale Steuergerät eine Druckinformation, die es ermöglicht, mit einem Rechner die momentane Förderleistung der Pumpe zu ermitteln, um beispielsweise die Förderleistung bzw. die Förderrate auf einen bestimmten Werkt zu regeln. Der Sensor 33 erkennt auch einen Druckstau bzw. ein örtliches Festsaugen, was dann als abnorme Betriebsbedingung registriert wird. Der Sensor 33 befindet sich mit Abstand unter dem Schirm 24, so dass der Schirm verhindert, dass Fremdkörper sich von außen gegen den Sensor legen. Der Sensor 33 ermittelt als Außendruck stets den wahren Außendruck, der den Pumpenring 23 umgibt. Der Sensor ist unter dem Schirm 24 in demselben Kompartiment angeordnet wie die Druckströmöffnung 17, so dass beide dem gleichen Druck ausgesetzt sind. Daher kann ein Ansaugen oder Festsaugen der Pumpe von dem Sensor erkannt werden und sensorbasiert zu einer Regulierung der Förderleistung der Pumpe benutzt werden.

Der Druck des Drucksensors 33 kann nicht nur dazu benutzt werden, den Pumpenbetrieb zu überwachen und zu regeln sondern auch dazu, die ordnungsgemäße Plazierung der Pumpe im Herzen festzustellen.

Entlang der Kanüle 13 erstreckt sich ein Druckübertragungsschlauch 32, der am distalen Ende 34 (Fig. 1) offen ist. Der Druckübertragungsschlauch 32 ist in das Gehäuse des Antriebsteils 11 eingebettet und er setzt sich weiter in dem Katheter 16 fort (Fig. 2). Sein extrakorporales Ende ist an ein Druckmessgerät angeschlossen, das den Druck auswertet.

Der Schirm 24 umgibt den Antriebsteil 11 und den Pumpenteil 12 mit einem radialen Abstand von 1 bis 2 mm. Der Schirm 24 bildet den Außenmantel der Blutpumpe 10. Sein Außendurchmesser beträgt, etwa 8 mm und seine rigide Länge beträgt etwa 40 mm. Der Schirm 24 besteht aus dünnem Stahl, bei dem die Einlassschlitze 26, 27 durch Laserschneidverfahren ausgeschnitten sind.

Das anhand der Fign. 3 bis 4 beschriebene Ausführungsbeispiel der Blutpumpe reduziert durch den die Durchströmöffnungen 17 umgebenden Schirm 24 die Neigung zum Ansaugen an dem leicht zu deformierenden Material des rechten Atriums. Beschädigungen der Tricuspidalklappe TK und des Klappenapparates mit der Folge der Gefahr des Pumpenstillstands werden vermieden. Der Drucksensor 33 ist freiliegend unter dem Schirm 24 angeordnet. Er kann nicht von-dem Ansauggebiet getrennt werden, so dass' auch ein eventuelles Festsaugen der Blutpumpe durch den Sensor erkannt wird. Gemäß Fig.. 2 wird der Blutstrom, der radial durch die Einlassschlitze 26, 27 eintritt, zunächst in axialer Richtung umgelenkt, bevor er die Durchströmöffnung 17 radial passiert.

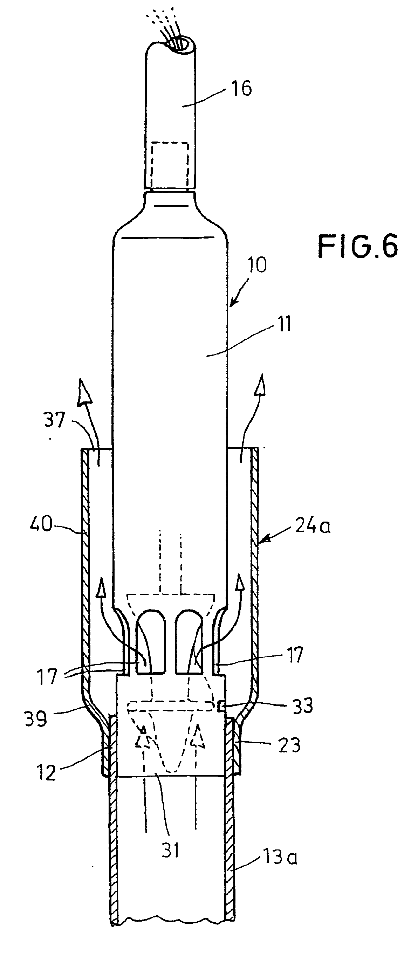

In den Fig. 5 und 6 ist ein Ausführungsbeispiel der Blutpumpe für die Förderung vom rechten Ventrikel RV in die Pulmonalarterie PA dargestellt. Die Blutpumpe 10 fördert hier in Rückwärtsrichtung, also zum Katheter 16 hin. Die Kanüle 13a, die sich an den Antriebsteil 11 anschließt, ist in diesem Fall gerade. Am freien Ende der Kanüle 13a befindet sich die Ansaugöffnung 35. Die Pumpe wird durch eine Inzisionsstelle 36 am oder unterhalb vom Abzweig der Pulmonalarterie PA in die Pulmonalarterie eingeführt. Der Abstand der Inzisionsstelle 36 von der Pulmonalklappe PK ist in vielen Fällen relativ kurz. Die Blutpumpe 10 sollte vollständig innerhalb der Pulmonalarterie PA untergebracht werden, wobei nur die Kanüle 13a durch die Pulmonalklappe PK hindurchragt. Durch das Vorhandensein des Schirmes 24a an der Blutpumpe 10 besteht die Möglichkeit, die Blutpumpe gewissermaßen zu verkürzen, wobei der Schirm 24a mit einem Teil der Blutpumpenlänge durch die Pulmonalklappe hindurchragt und die Auslassöffnung 37 sich etwa in der Mitte der Länge des Antriebsteils 11 befindet. Dadurch wird die rigide Länge der Pumpe, die in der Pulmonalarterie untergebracht wird, erheblich verkürzt.

Der Aufbau der Blutpumpe nach Fig. 5 ist in Fig. 6 im einzelnen dargestellt. Der Schirm 24a sitzt abdichtend auf dem Umfang des Pumpenringes 23 bzw. auf der Wand der mit dem Pumpenring verbundenen Kanüle 13a. Der Schirm 24a weist einen Übergang 39 auf, in dem er sich auf den Durchmesser eines zylindrischen Abschnitts 40 erweitert, welcher sich über den Antriebsteil 11 bis etwa zur Mitte der Länge des Antriebsteils erstreckt. Der zylindrische Abschnitt 40 endet in einer ringförmigen Auslassöffnung 37. Das Blut strömt aus der Kanüle 13a durch die Endöffnung 31 des Pumpenrings 23, in welchem sich das rotierende Pumpenrad befindet. Das Blut strömt dann weiter durch die seitlichen Durchströmöffnungen in das Innere des geschlossenwandigen Schirmes 24a. Der Schirm 24a ist nur an der axial gerichteten Auslassöffnung 37 offen. Der Blutstrom, der aus der Durchströmöffnung 17 radial austritt, wird von dem Schirm 24a in axiale Richtung umgelenkt. Da sich die Auslassöffnung nicht am vorderen Ende der Blutpumpe 10 sondern in deren Mittelbereich befindet, ist die operative Baulänge der Pumpe verkürzt.

Antriebsteil 11 und Pumpenteil 12 haben etwa gleichen Durchmesser. Der Schirm 24a umgibt den Antriebsteil 11 mit radialem Abstand.

Auch bei dem Ausführungsbeispiel nach Fig. 6 ist ein Drucksensor 33 am Pumpenring 23 vorgesehen. Der Drucksensor 33 ist ein Differenzdrucksensor, der in einer Öffnung des Pumpenrings angeordnet ist. Die innere Sensorfläche ist dem Innendruck des Pumpenteils ausgesetzt und die äußere Sensorfläche steht mit dem Innenraum des Schirms 24a in Verbindung und ist somit dem Auslassdruck der Pumpe ausgesetzt.

Die Blutpumpe 10 der beiden beschriebenen Ausführungsbeispiele sind grundsätzlich im Aufbau einander gleich. Sie unterscheiden sich durch die unterschiedlichen Förderrichtungen, die durch entsprechende elektrische Steuerung und Ausführung des Pumpenteils realisiert werden können.

## Patentansprüche

1. Intrakardiale Blutpumpe mit einem einen Motor (19) enthaltenden Antriebsteil (11), einem Pumpenteil (12), der ein von dem Motor (19) angetriebenes Pumpenrad (22) enthält, wobei der Blutstrom außen am Antriebsteil entlangströmt und zwischen dem Antriebsteil (11) und dem Pumpenteil (12) mindestens eine seitliche Durchströmöffnung (17) vorgesehen ist,
**dadurch gekennzeichnet,**
**dass** die Durchströmöffnung (17) mit einem Schirm (24,24a) bedeckt ist, der sich über die gesamte Länge der Durchströmöffnung erstreckt und den die Durchströmöffnung passierenden Blutstrom umlenkt.

2. Blutpumpe nach Anspruch 1, **dadurch gekennzeichnet, dass** die Durchströmöffnung (17) eine Einströmöffnung ist und der Schirm (24) an seinem Umfang mindestens eine Einlassöffnung (26, 27) aufweist.

3. Blutpumpe nach Anspruch 2, **dadurch gekennzeichnet, dass** der Schirm(24) mindestens eine Einlassöffnung (26) über dem Pumpenteil (12) aufweist.

4. Blutpumpe nach Anspruch 2 oder 3, **dadurch gekennzeichnet, dass** der Schirm (24) mindestens eine Einlassöffnung (27) über dem Antriebsteil (11) aufweist.

5. Blutpumpe nach einem der Ansprüche 1 - 4, **dadurch gekennzeichnet, dass** die Durchströmöffnung (17) eine Ausströmöffnung ist und der Schirm (24a) eine Auslassöffnung (37) um den Antriebsteil (11) aufweist.

6. Blutpumpe nach Anspruch 5, **dadurch gekennzeichnet, dass** der Schirm (24a) ein Rohr bildet, das an einem Ende abdichtend mit dem Pumpenteil (12) verbunden ist und dessen anderes Ende den Antriebsteil (11) mit radialem Abstand umgibt.

7. Blutpumpe nach einem der Ansprüche 1- 6, **dadurch gekennzeichnet, dass** am Pumpenteil (12) ein Drucksensor (33) vorgesehen ist, der von dem Schirm (24, 24a) überragt wird.

8. Blutpumpe nach Anspruch 7, **dadurch gekennzeichnet, dass** die Durchströmöffnung (17) und der Drucksensor (33) unter dem Schirm (24) druckmäßig untrennbar gekoppelt sind.

9. Blutpumpe nach einem der Ansprüche 1 - 7 oder 8, **dadurch gekennzeichnet, dass** der Pumpenteil (12) mit einer flexiblen Kanüle (13, 13a) verlängert ist.

10. Blutpumpe nach Anspruch 9, **dadurch gekennzeichnet, dass** längs der Kanüle (13) ein Druckübertragungsschlauch (32) verläuft, der mit einem mit dem Antriebsteil (11) verbundenen Katheter (16) in Verbindung steht.

11. Blutpumpe nach einem der Ansprüche 1 - 10, **dadurch gekennzeichnet, dass** der Schirm (24, 24a) auswechselbar befestigt ist.

12. Blutpumpe nach einem der Ansprüche 1 - 11, **dadurch gekennzeichnet, dass** der Durchmesser des Schirms (24, 24a) an keiner Stelle größer ist als 10 mm.

## Claims

1. Intracardiac blood pump comprising a drive portion (11) including a motor (19), a pump portion (12) including a pump wheel (22) driven by the motor (19), the blood stream flowing along the outside of the drive portion and at least one lateral flow opening (17) being provided between the drive portion (11) and the pump portion (12),
**characterized in**
**that** the flow opening (17) is covered by a screen (24,24a) extending over the entire length of the flow opening and diverting the blood stream passing the flow opening.

2. Blood pump according to claim 1, **characterized in that** the flow opening (17) is an inflow opening and the screen (24) comprises at least one inlet opening (26,27) at its circumference.

3. Blood pump according to claim 2, **characterized in that** the screen (24) comprises at least one inlet opening (26) above the pump portion (12).

4. Blood pump according to claim 2 or 3, **characterized in that** the screen (24) comprises at least one inlet opening (27) above the drive portion (11).

5. Blood pump according to one of claims 1-4, **characterized in that** the flow opening (17) is an outflow opening and the screen (24a) comprises an outlet opening (37) around the drive portion (11).

6. Blood pump according to claim 5, **characterized in that** the screen (24a) forms a tube that is sealingly connected with the pump portion (11) at one end thereof and the other end of which surrounds the drive portion (11) at a radial distance.

7. Blood pump according to one of claims 1 - 6, **characterized in that** a pressure sensor (33) is provided at the pump portion (12), above which the screen (24,24a) rises.

8. Blood pump according to claim 7, **characterized in that** the flow opening (17) and the pressure sensor (33) under the screen (24) are inseparably coupled to each other in terms of pressure.

9. Blood pump according to one of claims 1 - 7 or 8, **characterized in that** the pump portion (12) is extended by a flexible canula (13,13a).

10. Blood pump according to claim 9, **characterized in that** a pressure transmission hose (32) extends along the canula (13), which communicates with a catheter (16) connected with the drive portion (11).

11. Blood pump according to one of claims 1 - 10, **characterized in that** the screen (24,24a) is mounted exchangeably.

12. Blood pump according to one of claims 1 -11, **characterized in that** it is configured as an intravascular blood pump, the diameter of the screen (24,24a) being nowhere larger than 10 mm.

## Revendications

1. Pompe à sang intracardiaque avec une partie d'entraînement (11) contenant un moteur (19), une partie de pompage (12) contenant une roue de pompe (22) entraînée par le moteur (19), le flux sanguin s'écoulant à l'extérieur le long de la partie d'entraînement et au moins un orifice latéral de circulation (17) étant prévu entre la partie d'entraînement (11) et la partie de pompage (12),
**caractérisée en ce que** l'orifice de circulation (17) est recouvert d'un écran (24, 24a) qui s'étend sur toute la longueur de l'orifice de circulation et qui dévie le flux sanguin passant à travers l'orifice de circulation.

2. Pompe à sang intracardiaque selon la revendication 1, **caractérisée en ce que** l'orifice de circulation (17) est un orifice d'admission et **en ce que** l'écran (24) comporte au moins un orifice d'entrée (26, 27) sur sa périphérie.

3. Pompe à sang intracardiaque selon la revendication 2, **caractérisée en ce que** l'écran (24) comporte au moins un orifice d'entrée (26) sur la partie de pompage (12).

4. Pompe à sang intracardiaque selon la revendication 2 ou 3, **caractérisée en ce que** l'écran (24) comporte au moins un orifice d'entrée (27) sur la partie d'entraînement (11).

5. Pompe à sang selon l'une des revendications 1 à 4, **caractérisée en ce que** l'orifice de circulation (17) est un orifice d'évacuation et **en ce que** l'écran (24a) comporte une ouverture de sortie (37) autour de la partie d'entraînement (11).

6. Pompe à sang intracardiaque selon la revendication 5, **caractérisée en ce que** l'écran (24a) forme un tube, qui à une extrémité est relié en assurant l'étanchéité avec la partie de pompage (12) et dont l'autre extrémité entoure la partie d'entraînement (11) avec un écartement radial.

7. Pompe à sang intracardiaque selon l'une quelconque des revendications 1 à 6, **caractérisée en ce que** sur la partie de pompage (12), on a prévu un capteur de pression (33) sur lequel saillit l'écran (24, 24a).

8. Pompe à sang intracardiaque selon la revendication 7, **caractérisée en ce que** l'orifice de circulation (17) et le capteur de pression (33) sont couplés de façon indétachable au niveau pression sous l'écran (24).

9. Pompe à sang intracardiaque selon l'une quelconque des revendications 1 à 7 ou 8, **caractérisée en ce que** la partie de pompage (12) est prolongée par une canule flexible (13, 13a).

10. Pompe à sang intracardiaque selon la revendication 9, **caractérisée en ce qu'**un flexible transmetteur de pression (32), qui est en liaison avec un cathéter (16) relié avec la partie d'entraînement (11) s'étend le long de la canule (13).

11. Pompe à sang intracardiaque selon l'une quelconque des revendications 1 à 10, **caractérisée en ce que** l'écran (24, 24a) est fixé de façon interchangeable.

12. Pompe à sang intracardiaque selon l'une quelconque des revendications 1 à 11, **caractérisée en ce qu'**à aucun endroit, le diamètre de l'écran (24, 24a) n'est supérieur à 10 mm.
